Europäisches Patentamt

European Patent Office

Office européen des brevets

(11) Veröffentlichungsnummer: **0 503 418 A1**

# (12) EUROPÄISCHE PATENTANMELDUNG

(21) Anmeldenummer: **92103533.3**

(51) Int. Cl.5: **A61J 1/16**

(22) Anmeldetag: **02.03.92**

(30) Priorität: **08.03.91 DE 9102789 U**

(43) Veröffentlichungstag der Anmeldung:
**16.09.92 Patentblatt 92/38**

(84) Benannte Vertragsstaaten:
**AT BE CH DE DK ES FR GB GR IT LI LU NL PT SE**

(71) Anmelder: **HOECHST AKTIENGESELLSCHAFT**
**Postfach 80 03 20**
**W-6230 Frankfurt am Main 80(DE)**

(72) Erfinder: **Pajunk, Horst**
**Am Holzplatz 5-9**
**W-7716 Geisingen(DE)**
Erfinder: **Pajunk, Heinrich**
**Am Holzplatz 5-9**
**W-7716 Geisingen(DE)**
Erfinder: **Loos, Hans-Joachim**
**An der Schleuse 5**
**W-6095 Ginsheim-Gustavsburg(DE)**
Erfinder: **Ziegert, Günter**
**Loreleistrasse 34**
**W-6230 Frankfurt am Main 80(DE)**

(54) **Aufhängevorrichtung für Infusionsflaschen.**

(57) Bei der Aufhängevorrichtung für Infusionsflaschen mit 50 bis 1000 ml Inhalt bestehend aus einem Ring (7) mit Führungskanal (8), in dem ein Bügel (1) gleitend angeordnet ist, weist der Bügel (1) an einem Ende eine Aufhängeeinrichtung (3) und am anderen Ende einen Zentrierring (6) auf. Der Zentrierring 6 ist konzentrisch zum Ring (7) positioniert und der Ring (7) ist mit 2 parallel zueinander angeordneten Zentrierfedern (10) versehen.

Fig.

Die Erfindung betrifft eine universel verwendbare Aufhängevorrichtung für Infusionsflaschen mit 50 bis 1000 ml Inhalt, durch die die bekannten Einmalaufhängevorrichtungen ersetzt werden sollen.

Die Aufgabe wird durch eine Aufhängevorrichtung gelöst, die aus einem Ring mit Führungskanal besteht, in dem ein Bügel gleitend angeordnet ist, der Bügel an einem Ende eine Aufhängeeinrichtung und am anderen Ende einen Zentrierring aufweist, der konzentrisch zum Ring positioniert ist, und der Ring mit 2 parallel zu einander angeordneten Zentrierfedern versehen ist.

Der Bügel kann geteilt sein, wobei das Ende des einen Teiles mit einem Langloch in Richtung des Bügels und das andere Ende mit einer Stellschraube versehen ist.

Die Vorteile der Aufhängevorrichtung sind im wesentlichen in ihrer universellen Anwendbarkeit zu sehen. Flaschen von 50 bis 1000 ml Inhalt können gleichsam aufgenommen werden.

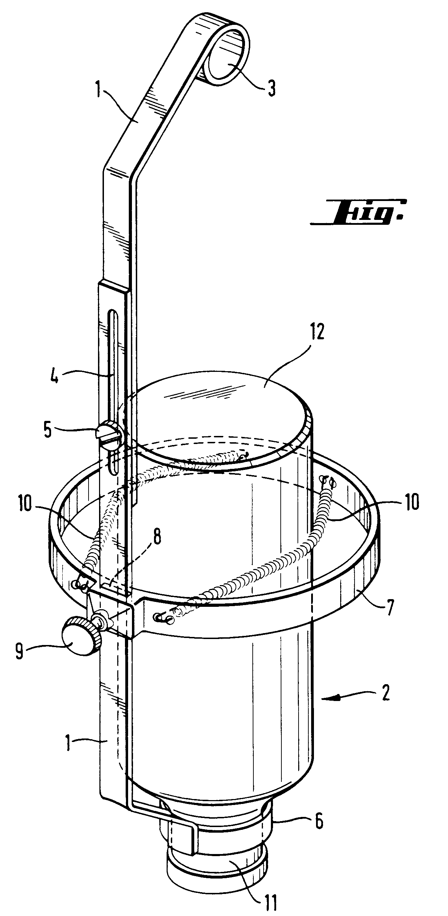

Die Erfindung wird in der Figur anhand eines Ausführungsbeispiels näher erläutert:
Ein Ring 7 ist mit einem Führungskanal 8 versehen, in dem ein Bügel 1 gleitet. Zum Feststellen des Bügels 1 ist der Führungskanal 8 mit einer Stellschraube 9 versehen. An einem Ende weist der Bügel eine Aufhängevorrichtung (Öse, Haken) 3 auf, an seinem anderen Ende einen Zentrierring 6, der konzentrisch zum Ring 7 positioniert ist. Der Zentrierring 6 nimmt den Flaschenhals 11, der Ring 7 den Flaschenkörper 12 auf. Die Infusionsflasche 2 wird im Ring 7 durch Zentrierfedern 10 und durch den Zentrierring 6 gehalten und zentriert. Der Bügel kann geteilt sein und das Ende eines Teiles mit einem Langloch 4 und das Ende des anderenTeiles mit einer Stellschraube 5 versehen sein.

## Patentansprüche

1.  Aufhängevorrichtung für Infusionsflaschen mit 50 bis 1000 ml Inhalt bestehend aus einem Ring (7) mit Führungskanal (8), in dem ein Bügel (1) gleitend angeordnet ist, der Bügel (1) an einem Ende eine Aufhängeeinrichtung (3) und am anderen Ende einen Zentrierring (6) aufweist, der konzentrisch zum Ring (7) positioniert ist, und der Ring (7) mit 2 parallel zueinander angeordneten Zentrierfedern (10) versehen ist.

2.  Aufhängevorrichtung nach Anspruch 1, dadurch gekennzeichnet, daß der Bügel (1) geteilt ist, wobei das Ende des einen Teiles mit einem Langloch (4) in Richtung des Bügels und das andere Ende mit einer Stellschraube (5) versehen ist.

Fig.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | FR-A-2 586 567 (DE RUYTER) <br> * Seite 2, Zeile 4 - Zeile 23; Abbildungen * <br> --- | 1 | A61J1/16 |
| A | FR-A-1 539 884 (LABORATOIRES ROBERT ET CARRIERE) <br> * Ansprüche; Abbildungen * <br> --- | 1 | |
| A | US-A-1 820 420 (AERICK) <br> * Seite 1, Zeile 54 - Zeile 59; Abbildung * <br> --- | 1 | |
| A | FR-A-2 568 771 (CHIARADIA) <br> * Anspruch; Abbildung 1 * <br> --- | 1 | |
| A | US-A-2 391 264 (NICKELSON) <br> * Spalte 2, Zeile 29 - Zeile 38; Abbildungen * <br> ----- | 1,2 | |

**RECHERCHIERTE SACHGEBIETE (Int. Cl.5 )**

A61G
A61J
A61M
B65D

Der vorliegende Recherchenbericht wurde für alle Patentansprüche erstellt

| Recherchenort | Abschlußdatum der Recherche | Prüfer |
|---|---|---|
| DEN HAAG | 14 APRIL 1992 | GODOT T. |